Europäisches Patentamt

European Patent Office

Office européen des brevets

(11) Publication number: **0 460 340 A1**

## EUROPEAN PATENT APPLICATION

(21) Application number: 90830531.1

(51) Int. Cl.5: **A61M 16/01**

(22) Date of filing: **19.11.90**

(30) Priority: **31.05.90 IT 353090**

(43) Date of publication of application:
**11.12.91 Bulletin 91/50**

(84) Designated Contracting States:
**AT BE CH DE DK ES FR GB GR LI NL SE**

(71) Applicant: SIARE HOSPITAL SUPPLIES S.r.l.
Via Lunga, 12/E

I-40056 Crespellano (Bologna)(IT)

(72) Inventor: **Preziosa, Giuseppe**
**Via Raibolini, 58/8**
**I-40069 Zola Predosa (Bologna)(IT)**

(74) Representative: **Pederzini, Paolo**
**c/o BUGNION S.p.A. Via dei Mille, 19**
**I-40121 Bologna(IT)**

(54) A system for verifying the supply of gases to surgery appliances, in particular to anaesthetizing apparatus.

(57) The system permits of ensuring that no switch has occurred in the two pressurized gas supplies to surgery equipment, and typically to anaesthetizing apparatus, by reducing the supply pressure of one gas to a value lower than that of the other gas; the two inlet pressures are verified by sensing elements (11, 12) built into or associated with the apparatus (1), and in the event that the resulting values fail to coincide with selected settings, the response triggers a warning indicator (19) and/or inhibits operation of the apparatus altogether.

FIG 1

The invention relates to a system for verifying the correct supply of gases to surgery appliances, and in particular to anaesthetizing apparatus.

Current methods in a conventional health service embrace the use of two different pressurized gases, suitably mixed together, in particular as a general anaesthetic during surgical operations.

The gases normally utilized for this purpose are oxygen and nitrous oxide, mixed one with the other in percentages that differ according to the stage of the operation.

At the outset, and during the operation, the gases are mixed in equal proportions; as the end of the operation approaches and the patient must reawaken, the quantity of nitrous oxide is gradually reduced, while the quantity of oxygen remains unaltered.

Nitrous oxide is a gas which, when mixed together with oxygen in correct proportion, causes loss of the senses; if administered to excess however, the consequences can be serious and even fatal.

The nitrous oxide and the oxygen are supplied to the place of use through two distribution circuits, installed side by side, connecting with respective bulk tanks sited remotely in safe surroundings.

Similarly, the coupling points to which the various surgery appliances are connected will be located one beside the other, as the gases are frequently used together and it would be inconvenient, even hazardous in some situations, for apparatus to be connected to the two supplies at points in a room distant from one another.

To prevent any possibility of wrong connections between the gas circuits and the appliance, the couplings used to connect the two appliance hoses to the supplies are totally dissimilar; thus, the oxygen hose cannot be coupled to the nitrous oxide supply, and neither can the nitrous oxide hose be connected to the oxygen supply.

Notwithstanding these precautions, it has happened that patients reawakening from general anaesthetic have suffered a drastic deterioration in physical conditions, terminating in decease.

Subsequent investigations into such cases, aimed at establishing whether or not the gas connections had managed in some way to become switched about, with the inevitable consequences that this would entail, revealed instead that the distribution circuits and the socket outlets had been free of defects.

The fault was discovered ultimately to lie in the appliance connecting hoses, which were duly fitted with dissimilar couplers and could not be connected incorrectly to the supply, but had indeed become switched, as the oxygen hose was found to carry the nitrous oxide coupler, and the nitrous oxide hose the oxygen coupler. Further investigation revealed that the connections had become switched not during construction and assembly at the works, where such an error would not be committed by skilled fitters, but during subsequent servicing operations that had included overhaul and replacement of the hoses.

Thus, a search was prompted for ways to avoid any such mortally hazardous inversion of the two gases.

The solutions proposed have been numerous, and in general costly, due to the chromatic and chemical similarity of the two gases.

Another factor, namely pressure, renders correct identification of the two gases in question still more problematic, as the selfsame gases, and oxygen in particular, are utilized by many anaesthetizing apparatus as an operating power source.

The object of the present invention is therefore to permit of identifying two gases, oxygen and nitrous oxide in particular, unequivocally and using simple and economical means.

The stated object is realized in the adoption of a verification system for gas supplies to surgery appliances as characterized in the appended claims.

The invention will now be described in detail, by way of example, with the aid of the accompanying drawings, in which:

- fig 1 illustrates the working principle of a control system according to the present invention;
- fig 2 illustrates the working principle of a control system according to the present invention, in an embodiment different to that of fig 1.

With reference to the drawings, a system according to the invention is designed for association with surgery appliances that make use of two pressurized gases, and in particular, anaesthetizing apparatus utilizing pressurized oxygen and nitrous oxide.

The apparatus is denoted 1 in its entirety, and in the case of the anaesthetics application considered by way of example, supplied by way of two inlet pipelines 2 and 3 with the two gases, oxygen and nitrous oxide respectively.

The apparatus 1 is positioned alongside a wall 4 of the surgery or hospital room, which affords two permanently installed couplers or supply points 5 and 6 connected to corresponding circuits 7 and 8 by which the respective gases, oxygen and nitrous oxide, are distributed around the building.

The inlet lines 2 and 3 likewise carry couplers 9 and 10 matched with, and only with, the relative supply points 5 and 6.

In a system according to the present invention, the pressure of one of the two gases, nitrous oxide in the case of the example illustrated, is reduced upstream of the relative inlet line 3 to a value different from that of the other gas.

Moreover, means 11 and 12 are associated with or integrated into the apparatus 1 by means of which to sense the pressure registering through the inlet line 3; these same means will be capable, moreover, of indicating wrong connections and/or inhibiting operation of the apparatus 1.

Where feasible, the reduction in pressure may occur upstream of the supply points 5 and 6, internally of the distribution circuit 7 or 8.

In the example illustrated, it is preferable in the interests of simplicity to effect the reduction not within the distribution circuit 8, but by means of a pressure-reducing valve 13 permanently associated with and indeed inseparable from the coupler 10 of the respective inlet line 3.

In a preferred embodiment, the pressure reducing valve 13 will have a fixed setting, in order to eliminate servicing requirements and preclude any possibility of tampering.

Accordingly, even in the event that the gas inlet line 3 needs renewing, the coupler 10 cannot be reattached directly but must be fitted by way of the pressure reducing valve 13, and the possibility of error is thus eliminated.

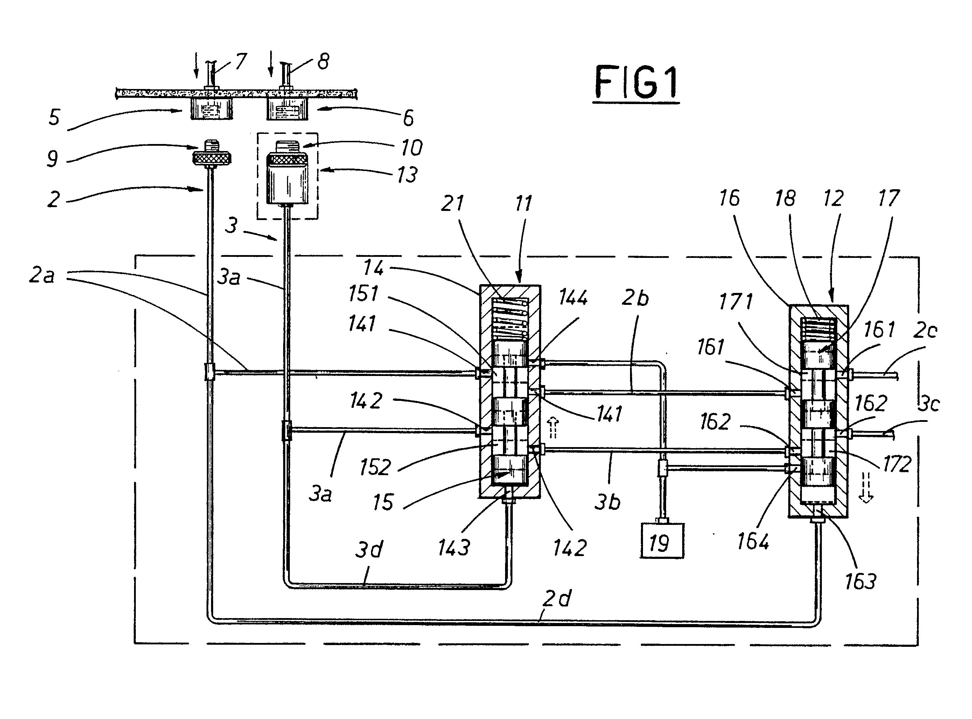

Pressure sensing means 11 and 12 might consist, as illustrated in fig 1, in a pair of pneumatically operated on-off valves installed in series on each of the inlet lines 2 and 3, such that each line is broken up into three successive sections denoted 2a-2b-2c and 3a-3b-3c respectively.

The single valves 11 and 12 comprise a housing 14 and 16 slidably accommodating a respective spool 15 and 17 capable of axial movement internally of and in fluid-tight association with the relative bore. Each valve housing 14 and 16 affords at least two pairs of radial holes 141-142 and 161-162 and one axial hole 143 and 163, respectively. The first sections 2a and 3a of the two inlet lines 2 and 3 connect with one of the paired holes 141 and 142 of the first valve 11, the second sections 2b and 3b connect the remaining hole 141 and 142 of each pair with one of the paired holes 161 and 162 of the second valve 12, and the third sections 2c and 3c connect the remaining hole 161 and 162 of each pair with the apparatus. The inlet lines 2 and 3 are also branched upstream of the valves 11 and 12 into further sections 2d and 3d connecting with the two axial holes 163 and 143, respectively.

Each of the spools 15 and 17 is biased toward an at-rest position by a corresponding spring 21, 18 located between the enclosed end of the housing 14, 16 and the spool itself. The springs 21 and 18 are calibrated to values such as will counterbalance a force exerted on the spools 15 and 17 by a pressure substantially equal to that registering through the one inlet line 2, whilst comfortably defeating a force exerted on the spools 15 and 17 by a pressure substantially equal to that which registers through the other inlet line 3 downstream of the pressure reducing valve 13. Thus, as discernible from fig 1, as long as no gas is supplied to the inlet lines 2 and 3, the spools 15 and 17 stay positioned against the relative axial holes 143 and 163, whereas with the supplies connected, the first spool 15 remains against the relative hole 143 due to the lower pressure of the nitrous oxide registering through the inlet line 3 whilst the second spool 17 will compress the relative spring 18 by reason of the comparatively greater pressure registering through the oxygen line 2.

The diameters and lands of the two spools 15 and 17 are arranged in such a way as to create two annular chambers in each of the housings 14 and 16, denoted 151, 152 and 171, 172 respectively. The length of the chambers 151, 152, 171 and 172, measured along the axis of the relative spool 15, 17, and their positions in relation to the spool axes, are such that the holes of each first pair 141 and 142 are connected when the relative spring 21 is extended, whilst the holes of each second pair 161 and 162 connect when the relative spring 18 is compressed, as illustrated in fig 1.

In short, the various sections 2a, 2b, 2c and 3a, 3b, 3c of the inlet lines 2 and 3 are connected one with the next only when pressure registers through the axial hole 143 of the first valve 11 at the lower value occasioned by the reducing valve 13, with pressure registering concurrently through the axial hole 163 of the second valve 12 at the supply value of the main circuit 7 (bold line in fig 1).

Any other combination of pressures will pilot one or both of the valves 11 and 12 to close, blocking off both the inlet lines 2 and 3 (phantom line in fig 1).

Clearly enough, the combination of pressures can occur only when the two inlet lines 2 and 3 are connected to the correct couplers 9 and 10, the latter by way of the pressure reducing valve 13.

If the lines 2 and 3 are switched, then the correct pressure combination is lost, the configuration of one or both valves 11 and 12 will be altered (see phantom arrow alongside each valve in fig 1), and both inlet lines 2 and 3 are blocked either by one or by both valves 11 and 12 (illustrated by phantom lines, fig 1).

To provide tangible evidence of wrong connections, each valve housing 14 and 16 might incorporate a further radial hole, denoted 144, 164 respectively, connected to a pneumatically operated visual and/or acoustic warning device 19. These holes 144 and 164 will be located in a position such as to connect with one of the two inlet holes 141 and 161 (or 142 and 162) by way of the relative annular chamber 151 and 171 (or 152 and 172) whenever the spools 15 and 17 shift to the limit positions opposite to those illustrated in fig 1, i.e. the normally extended spring 21 compressed by the one spool 15, and the normally compressed spring 18 extended against the other spool 17. Needless to say, it requires only one of the two spools 15 or 17 to move into the opposite limit position for the warning device 19 to operate. If no warning device 19 is installed, the radial holes of each pair 142, 142, 161 and 162 can be positioned coaxial one with another, or at least occupying a common diametral plane, whereas with the device in place, as in fig 1, the radial holes of each pair must be dissimilarly distanced from the relative axial hole 143 and 163 such that the spool 15 or 17 can either connect the two holes of the pair 141, 142, 161 and 162 one with another, or connect one hole of the pair with the hole 144 or 164 that activates the warning device 19.

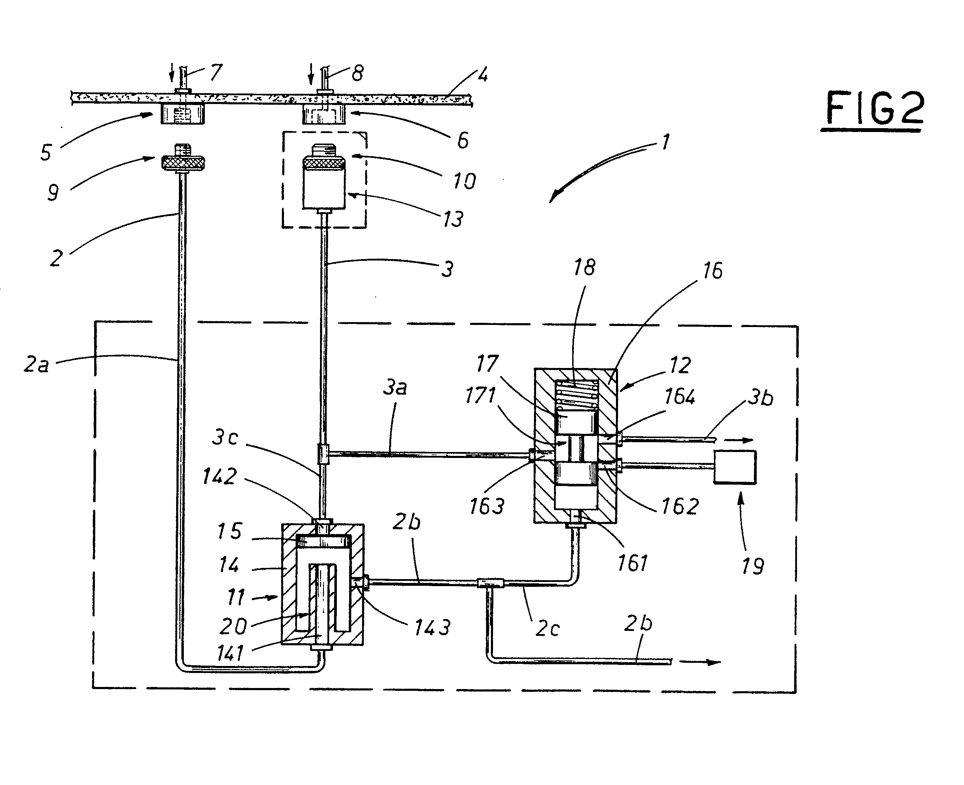

In the example of fig 2, pressure sensing means 11 and 12 consist respectively in a differential, or comparator valve and a pneumatically operated three way switching valve.

The comparator 11 is installed on the one inlet line 2, which thus comprises two sections 2a and 2b, respectively upstream and downstream.

The switching valve 12, comprising two input ports (one pilot) and two output ports, is installed on the remaining inlet line 3, which thus comprises two sections 3a and 3b in similar fashion.

In addition, this upstream section 3a constitutes one branch in a division of the inlet line 3, the remaining branch or section 3c connecting with the comparator 11 and counterbalancing the pressure through the upstream section 2a of the other line.

The comparator 11 comprises a housing 14 provided with two axial holes 141 and 142, one at each end, and a radial hole 143. The upstream section 2a and 3a of each inlet line connects with a corresponding axial hole 141 and 142 of the housing 14, whilst the downstream section denoted 2b connects with the radial hole 143. Internally, the housing 14 affords a tubular element 20 extending from one axial end hole 141 to a point beyond the radial hole 143, and accommodates a freely floating piston 15 coaxially disposed and capable of movement in response to the inlet pressure values which register through the two upstream sections 2a and 3a, shifting axially between the hole denoted 142 and the projecting end of the tubular element 20, against which it is able to seat and thus block the passage between the two remaining holes 141 and 143.

In the case of the switching valve 12, the housing 16 accommodates a spool 17 loaded axially against a spring 18.

The housing 16 affords an axial hole 161 at the end farthest from the spring 18, and three radial holes 162, 163 and 164 separated from the axial hole 161 by progressively increasing respective distances.

The middle, smaller diameter part of the spool 17 establishes an annular chamber 171 positioned in such a way as to encompass the intermediate radial hole 163, permanently, together with either one or other of the remaining radial holes 162 and 164.

The axial hole 161 connects with a third section 2c of the relative inlet line 2, which is branched from the downstream section 2b.

The spring 18 is calibrated to counterbalance a pressure not less than that registering through the section of inlet line 3 downstream of the reducing valve 13, and accordingly, unless the spool 17 is exposed to a pressure from the section of line denoted 2c, i.e. opposing the spring 18, greater than the output pressure setting of the reducing valve 13, compression will not occur.

The radial holes 162, 163 and 164 are connected, respectively, to a pneumatically operated visual and/or acoustic warning device 19 and to the upstream section 3a and the downstream section 3b of the lower pressure inlet 3.

It will been seen how, in this embodiment, when the inlet lines 2 and 3 are connected to the respective distribution circuits 7 and 8, oxygen enters the upstream section 2a of the relative inlet line 2 at normal supply pressure, whilst nitrous oxide enters the upstream section 3a of the remaining line 3 at the outlet pressure of the reducing valve 13. Given that the oxygen and nitrous oxide generally will be distributed substantially at the same pressure from the relative circuits 7 and 8, it follows that the nitrous oxide must enter the one inlet line 3 at a pressure lower, by reason of the reducing valve 13, than that of the oxygen entering the other line 2.

As a result of the different pressures registering internally of the comparator 11, the piston 15 shifts away from the projecting end of the tubular element 20 in such a way that the holes 141 and 143 of the inlet line can connect, and oxygen passes into the downstream section 2b at supply pressure.

Accordingly, with gas directed through the branched section 2c to the axial hole 161 of the switching valve 12 at this same pressure, the spring 18 will be compressed, and the radial holes 163 and 164 to the upstream and downstream sections 3a and 3b of the nitrous oxide inlet can thus connect. In this situation, the downstream sections 2b and 3b of the inlet lines 2 and 3 are supplied respectively with oxygen and nitrous oxide.

As regards the anaesthetizing apparatus 1 proper, operation is in no way affected by the fact that the nitrous oxide is supplied at a pressure lower than that of the oxygen. As long as surgery is in progress, the only gas that needs to be supplied at a substantial pressure and without interruption is the oxygen; thus, the fact that the nitrous oxide pressure happens to be lower than the pressure of the oxygen in no sense reflects an adverse affect on operation of the anaesthetizing apparatus 1.

Moreover, where the apparatus 1 is of a type that utilizes gas as a power source for its operation, the only gas of the two suitable for the purpose is oxygen, given that it must be exhausted following transmission of the stored energy.

In the course of routine servicing carried out on the apparatus 1, it may happen that the upstream sections 2a and 3a of the inlet lines need to be replaced, being flexible hoses located externally of the appliance and used to effect the connections to the distribution circuits 7 and 8, hence subject to a greater degree of wear; in the event that the couplers 9 and 10 should become switched, such that the nitrous oxide hose 3a is connected subsequently to the oxygen supply 7 and the oxygen hose 2a to the nitrous oxide supply 8, then operation of the apparatus 1 will be inhibited. With higher pressure registering through the upstream section 3a of the normally low pressure inlet, and therefore through the branched section 3c also, the axial hole 141 now receiving nitrous oxide from the other upstream section 2a will be blocked. Accordingly, the supply of nitrous oxide to the apparatus remains cut off.

Neither, with this configuration, does any pressure register through the branch 2c into the switching valve 12, with the result that the spring 18 can expand and thus cause the annular chamber 171 to encompass the hole 162 connecting with the warning device 19, which is duly activated by oxygen let in at supply pressure through the upstream section 3a of the normally low pressure inlet and the relative hole 163 of the housing 16. At this juncture, both the oxygen and the nitrous oxide supplies to the apparatus 1 are cut off and the warning device 19 is activated.

Two positive consequences are thus obtained: first, the assurance that lethal unmixed nitrous oxide cannot reach the patient, and second, the fact that staff are made aware of the wrong connection.

The stated object is thus comprehensively realized with a system according to the invention; moreover, a pressure reducing valve 13 made integral with the nitrous oxide coupler 10 ensures that no accidental connection error of any description can result in unmixed nitrous oxide reaching the patient.

Supposing in the worst imaginable situation that, following more extensive maintenance operations, the pressure comparator 11 and switching valve 12 were to be reconnected in such a way as to render their presence totally without effect, the reduced pressure of one of the gases in itself constitutes a safeguard to some degree. In effect, the moment that the aneasthetist begins reducing the flow rate and pressure of the nitrous oxide to reawaken the patient, this same reduction causes the apparatus 1 to shut off, given its reliance on the gases as a source of power; whilst it is true that this will cut off the supply of oxygen, the nitrous oxide is also safely isolated, whereupon anaesthetist and staff are left in no doubt as to the circumstances and can proceed with the appropriate measures.

Thus, in cases where oxygen only is used as a power source for operation of the apparatus, the sensing means and means which warn of wrong connections at the inlet lines 2 and 3 can be one and the same as the means driven by the oxygen.

The embodiment of fig 1 is more comprehensive than that of fig 2, given that in the event even of the coupler-and-reducing valve assembly 10-13 being replaced with a plain coupler, this is sufficient to upset the combination of pressures required to keep the inlet lines 2 and 3 open, as in fig 1.

With additional pressure sensing means 11 and 12, over and above the devices already built into the apparatus 1, pressure through the inlet lines 2 and 3 can be safety monitored even in appliances which make no use of oxygen as a power source.

Finally, the system according to the invention is especially economic and durable, as well as being maintenance-free.

**Claims**

1. A system for verifying the supply of gases to surgery appliances, in particular to anaesthetizing apparatus comprising two inlet pipelines (2, 3) in receipt of two gases under pressure, characterized,
   - in that the pressure of one of the two gases is reduced upstream of the inlet lines (2, 3) to a predetermined value different from that of the other gas; and
   - in that the apparatus (1) comprises pressure sensing means (11, 12) associated at least with the inlet line (3) in receipt of gas at the reduced pressure, designed to inhibit the operation of the apparatus (1) and/or to activate error detection means (19) on sensing a pressure value different to the predetermined value produced by the reduction upstream of the inlet lines (2, 3).

2. A system as in claim 1, associated with appliances by which one of the gases supplied under pressure is utilized also as a source of power in operation, wherein the pressure reduction is effected on the gas not utilized by the apparatus (1) as a power source.

3. A system as in claim 2, wherein the pressure of the gas subject to the reduction is lowered to a value less than the minimum pressure value required for the apparatus (1) to operate.

4. A system as in claim 1, associated with appliances of which the gas inlet pipelines (2, 3) are fitted with dissimilar couplers (9, 10) for connection to respective distribution circuits (7, 8), wherein one of the couplers is inseparably associated with a pressure reducing valve (13).

5. A system as in claim 1, associated with appliances of which the gas inlet pipelines (2, 3) are fitted with dissimilar couplers (9, 10) for connection to respective distribution circuits (7, 8), wherein one of the couplers is inseparably associated with a pressure reducing valve (13) of which the output setting is fixed.

6. A system as in claim 1, wherein pressure sensing means (11, 12) are embodied as two pneumatically operated on-off or switching valves installed in series along the gas inlet lines (2, 3), of which one valve (11) is piloted by one inlet line (3) and allows both inlet lines (2, 3) to remain open only on sensing a pressure less than or equal to the predetermined pressure of the gas subject to the pressure reduction, and the remaining valve (12) is piloted by the other inlet line (2) and allows both inlet lines (2, 3) to remain open only on sensing a pressure substantially equal to that of the gas not subject to the pressure reduction.

7. A system as in claim 6, wherein the pneumatically operated on-off or switching valves (11, 12) are connected to at least one pneumatically operated error detection device (19) in such a way as to activate the device on sensing a pressure value different to that of their respective settings.

8. A system as in claim 1, wherein pressure sensing means comprise:
   - a comparator element (11) of which the inputs (141, 142) are in receipt of gas respectively from an upstream section (2b) of the inlet line (2) not subject to the pressure reduction and an upstream section (3a) of the inlet line (3) subject to the pressure reduction, and of which the output (143) is connected to a downstream section (2b) of the line (2) not subject to the pressure reduction;
   - a pneumatically operated switching element (12) incorporating two inputs (163, 161) in receipt of gas respectively from the upstream section (3a) of the line (3) subject to the pressure reduction and from the downstream section (2b) of the line (2) not subject to the pressure reduction, and two outputs (164, 162) of which the first is normally open and connected to a downstream section (3b) of the line (3) subject to the pressure reduction and the second is normally closed and connected to the error detection means (19).

9. A system as in claim 1 for anaesthetizing apparatus in receipt of two pressurized gases of which one is oxygen and the other an anaesthetic gas, wherein the reduction in pressure is effected on the inlet line supplying the anaesthetic gas.

# FIG1

EP 0 460 340 A1

FIG2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB - A - 2 041 225 (THE MEDISHIELD C.) | 1-5,9 | A 61 M 16/01 |
| A | * Totality * | 6-8 | |
| A | GB - A - 1 149 767 (BRITISH OXYGEN CC.) * Totality; especially fig. 1,2; page 2, lines 34-68; page 3, lines 4-27 * | 1,2, 6-9 | |
| A | EP - A2 - 0 039 932 (DRÄGERWERK) * Totality; especially figure 1; page 6, line 5 - page 11, line 1; page 15, line 10 - page 16, line 19 * | 1-3,8, 9 | |
| A | DE - B2 - 2 351 718 (SOCIETE MINERVE) * Fig; column 4, lines 10-58 * | 1,2,6, 7,9 | |
| | | | TECHNICAL FIELDS SEARCHED (Int. Cl.5) |
| A | US - A - 4 191 952 (P.J. SCHREIBER et al.) * Fig. 2; column 2, line 30 - column 6, line 38 * | 1,7 | A 61 M |

The present search report has been drawn up for all claims

| Place of search | Date of completion of the search | Examiner |
|---|---|---|
| VIENNA | 13-09-1991 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)